# EUROPEAN PATENT SPECIFICATION

(11) **EP 3 756 344 B1**
(45) Date of publication and mention of the grant of the patent: **06.09.2023**
(21) Application number: 18907032.9
(22) Date of filing: 31.12.2018
(51) Int. Cl.: A61B 5/11, G16H 10/20, G16H 40/63, G16H 50/30, G06V 40/20, A61B 5/00

(54) **CONNECTED KIOSK FOR THE REAL-TIME ASSESSMENT OF FALLS RISK**
ANGESCHLOSSENER KIOSK ZUR ECHTZEITBEURTEILUNG DER STURZGEFAHR
KIOSQUE CONNECTÉ PERMETTANT L'ÉVALUATION EN TEMPS RÉEL D'UN RISQUE DE CHUTE

(30) Priority: 20.02.2018 US 201862632918 P; 28.12.2018 US 201816236050
(43) Date of publication of application: 30.12.2020
(73) Proprietor: Electronic Caregiver, Inc., Las Cruces, New Mexico 88001 (US)
(72) Inventor: DOHRMANN, Anthony, Las Cruces, New Mexico 88001 (US); CHASKO, Bryan J., Las Cruces, New Mexico 88001 (US); KEELEY, David W., Las Cruces, New Mexico 88001 (US)
(74) Representative: Lederer, Thomas L.
(86) International application number: PCT/US2018/068210
(87) International publication number: WO 2019/164585

(56) References cited:
- US-A1- 2010 124 737
- US-A1- 2013 204 545
- US-A1- 2014 148 733
- US-A1- 2015 359 467
- US-B1- 8 206 325
- US-B2- 7 612 681
- Marston Hannah R ET AL: "The design of a purpose-built exergame for fall prediction and prevention for older people", European review of aging and physical activity (vol. 12, article number: 13), 8 December 2015 (2015-12-08), pages 1-12, XP055848586, Germany DOI: 10.1186/s11556-015-0157-4 Retrieved from the Internet: URL:https://eurapa.biomedcentral.com/track /pdf/10.1186/s11556-015-0157-4.pdf [retrieved on 2021-10-06]
- Ejupi Andreas ET AL: "Kinect-Based Five-Times-Sit-to-Stand Test for Clinical and In-Home Assessment of Fall Risk in Older People", Gerontology (vol. 62), 28 May 2015 (2015-05-28), pages 118-124, XP055848593, Basel, Switzerland DOI: 10.1159/000381804 Retrieved from the Internet: URL:https://www.karger.com/Article/PDF/381 804 [retrieved on 2021-10-06]
- Festl Laura ET AL: "iStoppFalls: A Tutorial Concept and prototype Contents", , 30 March 2013 (2013-03-30), pages 1-36, XP055848585, Retrieved from the Internet: URL:https://hci-siegen.de/wp-uploads/2014/ 05/isf_tutorial_doku.pdf [retrieved on 2021-10-06]
- Wasenmüller Oliver ET AL: "Comparison of Kinect V1 and V2 Depth Images in Terms of Accuracy and Precision", Computer Vision - ACCV 2016 Workshops : ACCV 2016 International Workshops, Taipei, Taiwan, November 20-24, 2016, Revised Selected Papers, Part II, 16 March 2017 (2017-03-16), pages 1-12, XP055942856, Cham DOI: 10.1007/978-3-319-54427-4 ISBN: 978-3-319-54427-4 Retrieved from the Internet: URL:https://link.springer.com/content/pdf/ 10.1007/978-3-319-54427-4_3.pdf [retrieved on 2022-07-14]

## Description

### CROSS REFERENCE TO RELATED APPLICATIONS

This application claims the priority benefit of U.S. Provisional Patent Application Serial No. 62/632,918 filed on February 20, 2018 and titled "Connected Kiosk for the Real-Time Assessment of Falls Risk," and is related to U.S. Patent Application Serial No. 16/169,760 filed on October 24, 2018 and titled "Computing Devices with Improved Interactive Animated Conversational Interface Systems".

### FIELD OF THE TECHNOLOGY

Embodiments of the disclosure relate to a deployable fall risk assessment kiosk. In particular, the present disclosure relates to systems and methods providing real-time assessment of falls risk.

### BACKGROUND

The articles "The design of a purpose-built exergame for fall prediction and prevention for older people" (Marston Hannah R ET AL: European review of aging and physical activity, vol. 12, article number: 13, 8 December 2015) and "Kinect-Based Five-Times-Sit-to-Stand Test for Clinical and In-Home Assessment of Fall Risk in Older People" (Ejupi Andreas ET AL: Gerontology, vol. 62, 28 May 2015, pages 118-124) disclose together a fall risk assessment system involving a fall risk assessment system. The fall risk assessment is made by combining answers to questions relating to various domains as well as results to a plurality of physical assessments. Some of the physical assessments are assessed using a Microsoft Kinect^{™}.

The approaches described in this section could be pursued, but are not necessarily approaches that have previously been conceived or pursued. Therefore, unless otherwise indicated, it should not be assumed that any of the approaches described in this section qualify as prior art merely by virtue of their inclusion in this section.

Fall risk assessment has become an integral part of the provision of health care to aged Americans. This coupled with the fact that the rapidly aging population of the United States has a great desire to maintain independence has led to the identification of a number of risk factors associated with falling. Unfortunately, most risk factor assessments take place in the office of a certified medical professional or in a brick-and-mortar research laboratory. This results in three primary limitations associated with the current fall risk assessment paradigm: 1) there is a travel component related to an individual's ability to complete a comprehensive fall risk assessment; 2) the traditional hardware and software costs associated with comprehensive fall risk assessment are prohibitive to the widespread conduction of these assessments; and 3) due to the logistical difficulties associated with conducting comprehensive falls risk assessments, individuals are commonly tested only once or twice per year.

These problems associated with comprehensive fall risk assessment have yet to be fully solved by currently available systems. While evidence suggests that the known factors associated with increased risk in falling can be identified and corrected, most currently available methods of comprehensive fall risk assessment are not patient-centric. Additionally, current assessment systems are not mobile and do not incorporate multiple methods of fall risk screening.

### SUMMARY

The invention is defined by the appended claims.

According to some embodiments, the present technology is directed to a method for providing real-time assessment of falling risk, the method comprising: (a) providing a user with a survey comprising questions directed to eliciting information regarding assessing falls risk across a plurality of domains; (b) receiving the elicited information comprising answers to the questions that are indicative of a risk of falling of the user; (c) recording, via a depth camera system, depth video data of an activity of the user; (d) determining, based on the depth video data, at least one spatio-temporal gait characteristic associated with the activity performed by the user; (e) conducting a quantitative fall risk analysis based on the at least one spatio-temporal gait characteristic and the elicited information; and (f) displaying at least a portion of the quantitative fall risk analysis to the user.

In some embodiments, the present disclosure is directed to a system of one or more computers which can be configured to perform particular operations or actions by virtue of having software, firmware, hardware, or a combination thereof installed on the system that in operation causes or cause the system to perform actions and/or method steps as described herein. For example, embodiments may include a system for providing real-time assessment of falling risk, the system comprising: (a) a kiosk; (b) a tablet device communicatively coupled to the kiosk; (c) a depth camera system communicatively coupled to the kiosk; (d) at least one processor; and (e) a memory storing processor-executable instructions, wherein the at least one processor is configured to implement the following operations upon executing the processor-executable instructions: (i) providing a user with a survey comprising questions directed to eliciting information regarding assessing falls risk across a plurality of domains; (ii) receiving the elicited information comprising answers to the questions that are indicative of a risk of falling of the user; (iii) recording, via a depth camera system, depth video data of an activity of the user; (iv) determining, based on the depth video data, at least one spatio-temporal gait characteristic associated with the activity performed by the user; (v) conducting a quantitative fall risk analysis based on the at least one spatio-temporal gait characteristic and the elicited information; and (vi) displaying at least a portion of the quantitative fall risk analysis to the user.

In another embodiment, the present disclosure comprises a non-transitory computer readable medium having embodied thereon instructions being executable by at least one processor to perform a method for providing real-time assessment of falling risk, the method comprising: (a) providing a user with a survey comprising questions directed to eliciting information regarding assessing falls risk across a plurality of domains; (b) receiving the elicited information comprising answers to the questions that are indicative of a risk of falling of the user; (c) recording, via a depth camera system, depth video data of an activity of the user; (d) determining, based on the depth video data, at least one spatio-temporal gait characteristic associated with the activity performed by the user; (e) conducting a quantitative fall risk analysis based on the at least one spatio-temporal gait characteristic and the elicited information; and (f) displaying at least a portion of the quantitative fall risk analysis to the user.

### BRIEF DESCRIPTION OF THE DRAWINGS

The accompanying drawings, where like reference numerals refer to identical or functionally similar elements throughout the separate views, together with the detailed description below, are incorporated in and form part of the specification, and serve to further illustrate embodiments of concepts that include the claimed disclosure, and explain various principles and advantages of those embodiments.
FIG. 1 shows a high-level block diagram of an exemplary system configured to provide a comprehensive fall risk assessment, according to the subject disclosure.
FIG. 2 shows a high-level block diagram of an exemplary system for providing and processing a comprehensive survey, according to the subject disclosure.
FIGS. 3a-3c illustrate instances of an exemplary graphical user interface configured to facilitate receiving comprehensive survey responses, according to the subject disclosure.
FIG. 4 shows a high-level block diagram of an exemplary system for providing and processing gait analysis, according to the subject disclosure.
FIGS. 5a-5b illustrate exemplary graphical representations of a functional assessment of the gait analysis, according to the subject disclosure.
FIG. 6 illustrates an exemplary graphical user interface having functional movement analysis results, according to the subject disclosure.
FIG. 7 shows a high-level block diagram of an exemplary system configured to provide evidence-based recommendations based on comparative data, according to the subject disclosure.
FIG. 8 illustrates an exemplary graphical user interface having results of the comprehensive fall risk assessment.
FIG. 9 illustrates an exemplary graphical user interface configured to provide recommendations based on an environmental survey, according to the subject disclosure.
FIG. 10 illustrates an exemplary depiction of an electronic caregiver image (ECI) avatar configured to guide a user through the completion of the comprehensive fall risk assessment, according to the subject disclosure.
FIG. 11 illustrates an exemplary depiction of the ECI avatar providing representative examples of functional tests to be performed by a user, according to the subject disclosure.
FIG. 12 depicts a process flow diagram showing a method for providing a comprehensive fall risk assessment, according to the subject disclosure.
FIG. 13 illustrates an exemplary computer system that may be used to implement embodiments of the present disclosure.

### DETAILED DESCRIPTION

In the following description, for purposes of explanation, numerous specific details are set forth in order to provide a thorough understanding of the disclosure. It will be apparent, however, to one skilled in the art, that the disclosure may be practiced without these specific details. In other instances, structures and devices may be shown in block diagram form only in order to avoid obscuring the disclosure. It should be understood, that the disclosed embodiments are merely exemplary of the invention, which may be embodied in multiple forms. Those details disclosed herein are not to be interpreted in any form as limiting, but as the basis for the claims.

Various exemplary embodiments described and illustrated herein relate to a computing device configured to provide a patient-centric and mobile method of comprehensive fall risk assessment. The computing device may comprise a kiosk fall risk assessment system that performs a survey and gait analysis on individuals using a validated survey research instrument and a depth camera. Notably, the kiosk fall risk assessment system may utilize one or more interfaces, cameras, and sensors other than electrocardiograph (ECG). The completion of the survey and instructions for the gait analysis are provided to the user via a conversational interface system, as described in greater detail in related U.S. Patent Application Serial No. 16/169,760 filed on October 24, 2018 and titled "Computing Devices with Improved Interactive Animated Conversational Interface Systems". The gait analysis incorporates the conversational interface system, a depth camera, a tablet device, and algorithms and machine learning to calculate one or more gait characteristics of the individual and fall risk scores. The results of the comprehensive survey, the gait characteristics, and the fall risk scores may then be provided to the individual through the conversational interface and kiosk.

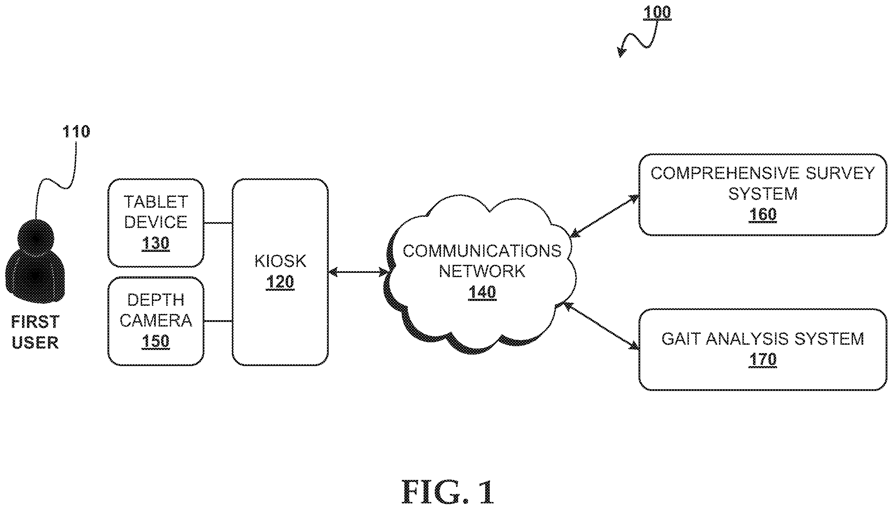

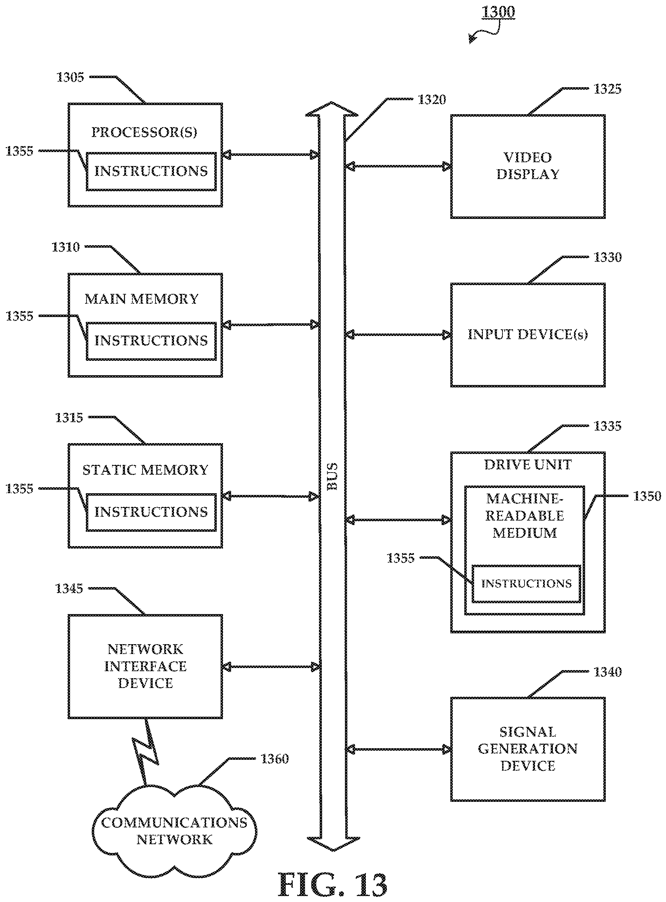

FIG. 1 depicts a kiosk fall risk assessment system 100 configured to provide a comprehensive fall risk assessment of a user. In various embodiments, the kiosk fall risk assessment system 100 includes a kiosk 120, a communications network 140, a comprehensive survey system 160, and a gait analysis system 170. The kiosk 120 may include or be coupled to a tablet device 130 and a depth camera system 150. A user 110 utilizes the kiosk system 120 through a tablet device 130 that is coupled to a communications network 140, such as the Internet or cellular network. An Electronic Caregiver system may be installed on the tablet device 130 configured to provide a comprehensive fall risk assessment for the user 110, as will be described in greater detail throughout the present disclosure. To be sure, the tablet device 130 and kiosk 120 may be replaced with, or take the form of, any suitable computing device, such as those described with respect to FIG. 13. The depth camera system 150 facilitates a functional movement assessment to measure one or more parameters of the user's movement. While not shown, it is to be understood that the kiosk fall risk assessment system 100 may further comprise any number or combination of sensors, including video camera systems, movement sensors, vibration sensors, or the like.

The comprehensive fall risk assessment is based on results from one or both of the comprehensive survey system 160 and the gait analysis system 170. The comprehensive survey system 160 and/or the gait analysis system may be cloud-based, or comprise one or more remote servers. It is to be understood that the one or more servers providing services for the comprehensive survey system 160 and the gait analysis system 170 may be the same remote servers, separate remote servers, or have one or more shared servers. Through an assessment of the comprehensive survey system 160 and/or the gait analysis system 170, the kiosk fall risk assessment system 100 facilitates providing the user 110 with clinically relevant data describing their overall risk of falling.

The communications network 140 may include a wireless or wire network, or a combination thereof. For example, the network may include one or more of the following: the Internet, local intranet, PAN (Personal Area Network), LAN (Local Area Network), WAN (Wide Area Network), MAN (Metropolitan Area Network), virtual private network (VPN), storage area network (SAN), frame relay connection, Advanced Intelligent Network (AIN) connection, synchronous optical network (SONET) connection, digital T1, T3, E1 or E3 line, Digital Data Service (DDS) connection, DSL (Digital Subscriber Line) connection, Ethernet connection, ISDN (Integrated Services Digital Network) line, dial-up port such as a V.90, V.34 or V.34bis analog modem connection, cable modem, ATM (Asynchronous Transfer Mode) connection, or an FDDI (Fiber Distributed Data Interface) or CDDI (Copper Distributed Data Interface) connection. Furthermore, the communications may also include links to any of a variety of wireless networks including, WAP (Wireless Application Protocol), GPRS (General Packet Radio Service), GSM (Global System for Mobile Communication), CDMA (Code Division Multiple Access) or TDMA (Time Division Multiple Access), cellular phone networks, GPS, CDPD (cellular digital packet data), RIM (Research in Motion, Limited) duplex paging network, Bluetooth radio, or an IEEE 802.11-based radio frequency network. The network can further include or interface with any one or more of the following: RS-232 serial connection, IEEE-1394 (Firewire) connection, Fiber Channel connection, IrDA (infrared) port, SCSI (Small Computer Systems Interface) connection, USB (Universal Serial Bus) connection, or other wired or wireless, digital or analog interface or connection, mesh or Digi^{®} networking.

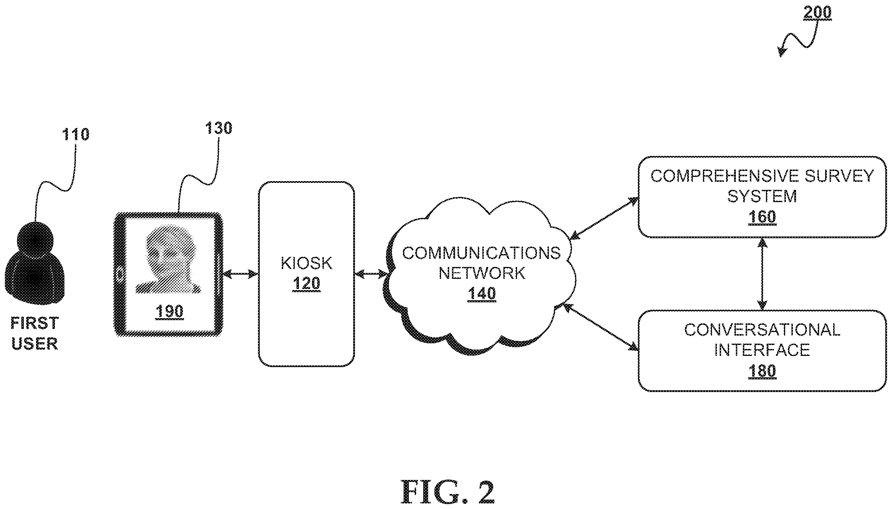

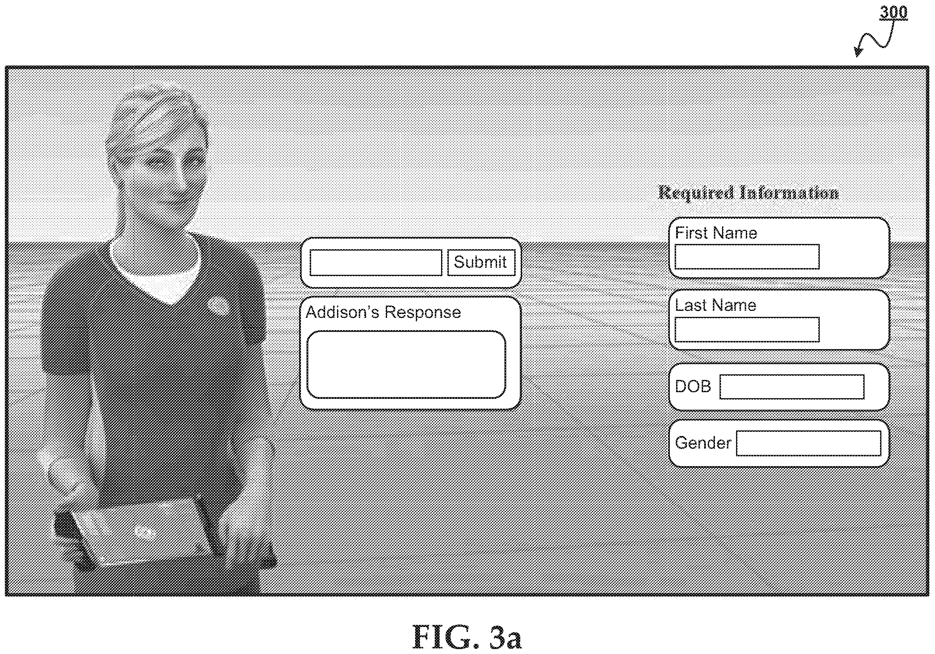

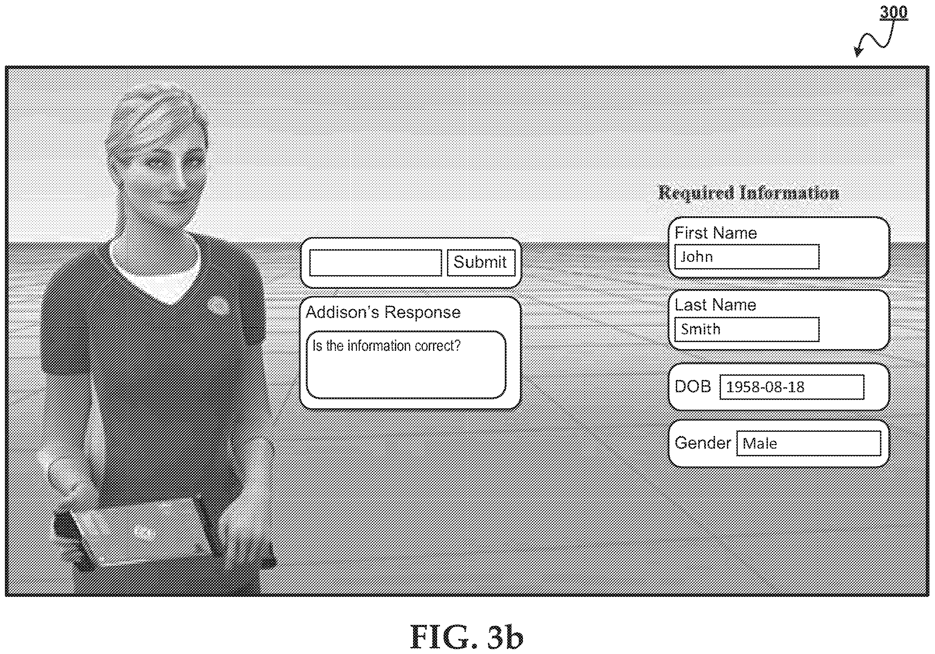

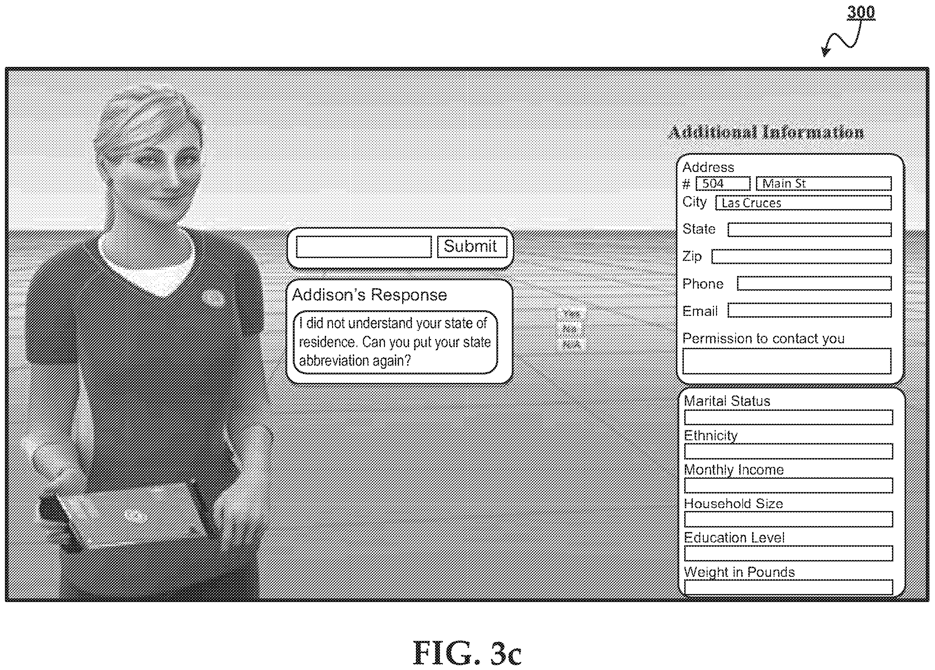

The comprehensive survey system 160 may utilize an interactive, conversational, text-based interaction and a three-dimensional Electronic Caregiver Image (ECI) avatar that allow a user to complete various forms using voice conversation and cloud-based talk-to-text technology. The comprehensive survey system 160 will be described in greater detail with respect to FIGS. 2, 3a, 3b, and 3c.

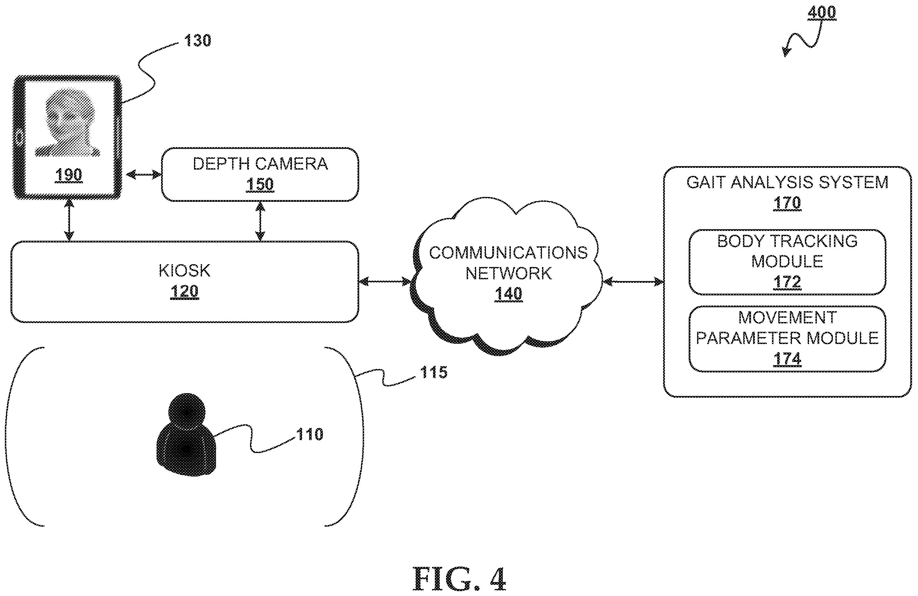

The gait analysis system 170 is configured to assess and quantify one or more spatio-temporal gait characteristics, and a capability for completion, of the user 110 from one or more functional assessments. Functional assessments may include physical actions performed by the user, such as walking, sitting, turning, etc. The gait analysis system 170 will be described in greater detail with respect to FIGS. 4, 5a, 5b, and 6.

FIG. 2 depicts a high-level block diagram of an exemplary system 200 for providing and processing a comprehensive survey using the comprehensive survey system 160. It is to be understood that the exemplary system 200 may be a portion of the kiosk fall risk assessment system 100 illustrated in FIG. 1. In various embodiments, the comprehensive survey system 160 is coupled with an electronic caregiver derived interactive animated conversational interface system having an interactive, conversational text-based communication system (referred to herein as a conversational interface) 180 and an Electronic Caregiver Image (ECI) avatar 190. Examples of the conversational interface 180 and the ECI avatar 190 are described in related U.S. Patent Application Serial No. 16/169,760 filed on October 24, 2018 and titled "Computing Devices with Improved Interactive Animated Conversational Interface Systems".

Through the exemplary system 200, the ECI avatar 190 engages with the user 110 to provide a survey comprising a series of questions directed to eliciting information regarding assessing falls risk across a plurality of domains, and receiving the elicited information comprising answers to the questions that are indicative of a risk of falling of the user. The ECI avatar 190 may communicate in multiple languages. The exemplary system 200 provides the user 110 with an option of selecting methods for data input, methods comprising either type-based data entry or voice communication data entry.

Following the user input of data, including the elicited information, the data may be transmitted from the tablet device 130, to the kiosk 120, through the communications network 140 to the conversational interface 180 and/or comprehensive survey system 160. The conversational interface 180 analyzes the data input and determines redundancy against data input errors. In response to determining an error in the data input, the conversational interface 180 causes feedback to be provided to the user 110 for correction of errors, for example by the ECI avatar 190. The conversational interface 180 may also transmit the input data to the comprehensive survey system 160, or inform the comprehensive survey system 160 of the results of the error determination.

To assess data for accuracy in real-time, the conversational interface 180 utilizes a catalogue of inputs to determine whether a data type input by the user 110 matches a defined catalogue data type. As such, through the use of cloud-based applications such as the conversational interface 180 and comprehensive survey system 160, the exemplary system 200 assesses the input data, determines whether errors exist in the input data, executes a continuation decision process, and provides a response to the user in real time. In general, the real time response may be provided to the user in a suitable period of time in which the user may wait, such as less than 1.0 second. Once data has been assessed for accuracy and all input data is entered, the comprehensive survey system 160 encrypts user input data and proceeds with transmitting the data to a cloud-based primary key design database for storage.

The exemplary system 200 also provides a web browser comprising the three-dimensional ECI avatar 190 for interactive communication with the user 110. This ECI avatar 190 provides the user with an interactive experience during which the user is guided through the completion of the comprehensive fall risk assessment. As the assessment is completed by the user, the ECI avatar 190 provides real-time feedback in conversational form in an effort to simplify and streamline the form completion by the user.

The ECI avatar 190 is displayed to the user 110 via a graphical user interface of the tablet device 130. The ECI avatar 190, via the tablet device 130, presents the series of questions to the user, the series of questions assessing falls risk across one or more domains. The domains, described in Table 1 below, are inclusive of the History, Medication, Environmental, Physical and Vision aspects of a user.

**Table 1. Domains of fall risk assessed by the comprehensive survey system incorporated into the kiosk fall risk assessment system and sample inquiry areas for each of the domains.**

| **Domain** | **Example Information Collected** |
|---|---|
| History | 1. Age |
| | 2. History of Falls |
| | 3. Assistive Device Use |
| | 4. Arthritis Diagnosis |
| Medication | 1. Medication List |
| | 2. Experiencing of Side Effects |
| | 3. Number of Pharmacists |
| | 4. Pharmacy Consult Frequency |
| Environmental | 1. Lighted Stairways from Top to Bottom |
| | 2. Floors Clear of Clutter |
| | 3. Non-Skid Rugs |
| Physical | 1. Extended Timed Up-and-Go |
| | 2. Functional Reach Test |
| Vision | 1. Wearing of Corrective Lenses as Prescribed |
| | 2. Vision Test within Last Year |
| | 3. Snellen Eye Score |

The kiosk 120 provides the user 110 with access to talk-to-text functionality. The kiosk 120 provides the user 110 access to cloud-based services comprising computing capacity for the provision of both voice and text communication using the ECI avatar 190. As such, upon initiation of the comprehensive survey system 160 by the user 110, the ECI avatar 190 facilitates requesting the data necessary for completion of the comprehensive survey by way of simple conversation.

In certain embodiments, the exemplary system 200 validates survey data input by the user 110. Once survey response data from the user 110 are received by the kiosk 120 and transmitted to the conversational interface 180 via communications network 140, the conversational interface 180 compares the data type (e.g. yes/no, words, numbers, etc.) of the input to predefined data types stored in a validation utility. Thus, the conversational interface 180 assesses the validity of data input types provided by the user 110. Upon determining the input is valid by the validation utility, the conversational interface 180 activates a progression decision program, which causes the kiosk 120 to move on to the next item contained in the comprehensive survey to be inquired of the user 110. Alternatively, upon determining the input is invalid by the validation utility, the progression decision program is not activated, and instead the conversational interface 180 triggers the comprehensive survey system 160 to inform the user 110 of the invalid data input.
The kiosk 120, via the tablet device 130 and ECI avatar 190, informs the user 110 that the previous data input is invalid and asks the survey question again.

FIGS. 3a-3c illustrate instances of an exemplary graphical user interface 300 configured to facilitate receiving comprehensive survey responses. FIGS. 3a-3c depict the various decision paths based on input data from the user. It is to be understood that the graphical user interface 300 may be a part of the tablet device 130 depicted in FIGS. 1-2, or another suitable display coupled to the kiosk 120.

FIG. 3a depicts an initial set of uncompleted form fields displayed to the user 110 on the tablet device 130. FIG. 3b depicts the presentation of input data to the user 110 on the tablet device 130 that has been determined to be valid by the conversational interface 180. FIG. 3c depicts an invalid data input response from the ECI avatar 190.

FIG. 4 illustrates an exemplary system 400 for providing and processing a gait analysis using the gait analysis system 170. The gait analysis system 170 may include a body tracking module 172 and a movement parameter module 174, as will be described in greater detail below. It is to be understood that the exemplary system 400 may be a portion of the kiosk fall assessment system 100 illustrated in FIG. 1. In various embodiments, the kiosk 120 is further configured to facilitate assessing and quantifying one or more spatio-temporal gait characteristics of the user 110, as well as the user's capability for completion of functional assessments. Several examples of the spatio-temporal gait characteristics are described in Table 2.

**Table 2. Functional movement parameters determined by the gait analysis system provided to user via the kiosk fall risk assessment system.**

| **Quantified Variable** | **Definition** |
|---|---|
| Gait Velocity | Linear distance traveled by the user per unit time. |
| Stride Length | Linear distance from points of successive foot strike of the same foot. |
| Step Length | Linear distance from point of foot strike for the contralateral foot. |
| Step Width | Linear distance in the frontal plane from point of foot strike for the contralateral foot. |
| Time in Stance | Percentage of the gait cycle during which the foot is in contact with the ground. |
| Time in Swing | Percentage of the gait cycle during which the foot is not in contact with the ground. |
| Time in Double Support | Percentage of the gait cycle during which both feet are in contact with the ground. |
| Stance time Variability | Average variability associated with the user stance time over the course of ten assessments. |
| Swing time Variability | Average variability associated with the user swing time over the course of ten assessments. |
| Timed Up-and-Go Test Duration | Duration (in seconds) it requires the user to complete functional assessment. |
| Timed Up-and-Go Test Turn Duration | Duration (in seconds) it requires the user to complete the turning portion of the functional assessment. |
| Timed Up-and-Go Test Stand Duration | Duration (in seconds) it requires the user to complete the sit-to-stand portion of the functional assessment. |
| Timed Up-and-Go Test Sit Duration | Duration (in seconds) it requires the user to complete the stand-to-sit portion of the functional assessment. |
| 30-second Chair Stand Repetitions | Number of sit-to-stand repetitions the user completes during test period (30 seconds). |
| 30-second Chair Stand Decay Rate | Rate of time decay associated with each repetition completed by the user during the testing period (30 seconds). |

To perform the gait analysis of a functional assessment, the exemplary system 400 utilizes the depth camera system 150 and the gait analysis system 170. The kiosk 120 initiates a functional assessment by inquiring of the readiness of the user 110. Following an acknowledgement of readiness by the user 110, the kiosk 120 initiates a video depth recording, via depth camera system 150, as the user walks and/or performs the recommended functional assessments. The user 110 performs the activity or activities necessary for the functional assessment as indicated by the kiosk 120 in the movement performance area 115. During performance of the functional test by the user 110, the kiosk 120 uses the depth camera system 150 to record a depth video of the activity.

The depth video is transmitted by the kiosk 120, either in real-time, at the completion of each activity, or the competition of the functional assessment, to the gait analysis system 170 via the communications network 140. The body tracking module 172 applies a body tracking algorithm to the depth video, including determining a position and an orientation of the user in space for the duration of the depth video data. The body tracking algorithm may include determining a location within the depth video of one or more limbs of the user 110, and tracking the position and orientation of the one or more limbs for each frame within the depth video data. The movement parameter module 174 may process the body tracking information produced by the body tracking module 172 to determine the one or more spatio-temporal gait characteristics of the user 110. For example, the movement parameter module 174 may determine the gait velocity of the user 110 by calculating the linear distance traveled by the user 110 divided by the length of time required for the user 110 to travel that distance. The one or more spatio-temporal gait characteristics may be stored in a database associated with the gait analysis system 170, the kiosk 120, the tablet device 130, or any combination thereof.

To be sure, while the gait analysis system 170, including the body tracking module 172 and movement parameter module 174, are shown as being remote from the kiosk 120 over communications network 140, embodiments of the present disclosure include having the gait analysis system 170 in the kiosk 120 or tablet device 130.

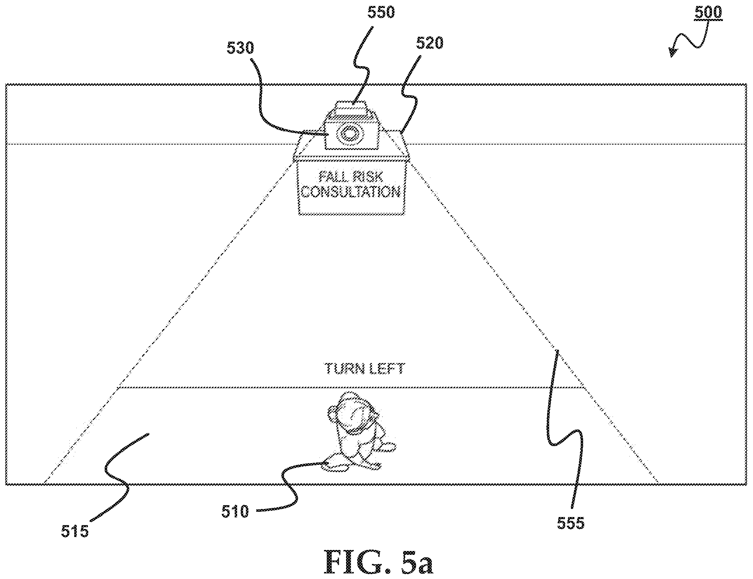

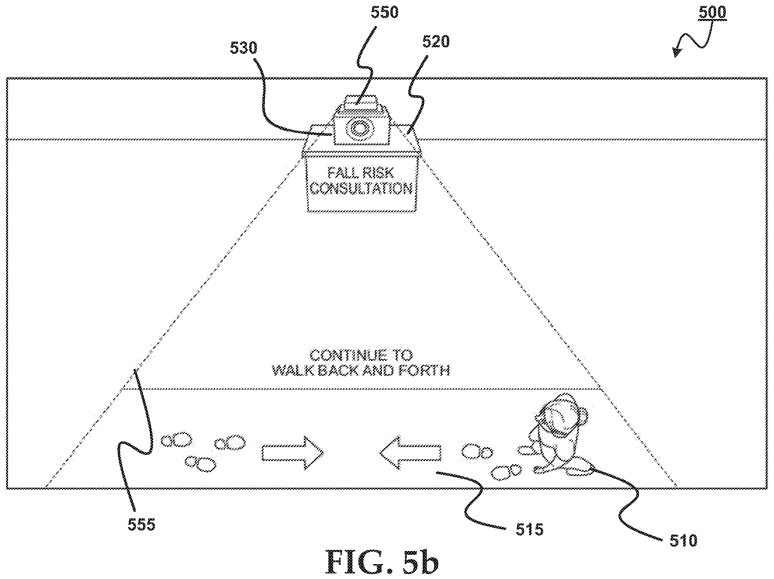

FIGS. 5a and 5b depict a graphical user interface 500 showing a representation of an activity of a functional assessment. The graphical user interface 500 may be presented to the user 110 via the tablet device 130 as shown in FIG. 4, for example. The representation may include a graphical depiction of the user 510, movement performance area 515, kiosk 520, tablet device 530, and depth camera system 550. The graphical user interface 500 may further depict a representative field of view 555 of the depth camera system 150 such that the user 110 may be informed as to the boundaries of the movement performance area 115. In particular, FIG. 5a illustrates an activity of a functional assessment involving the user 110 turning left from a stationary position. FIG. 5b illustrates another activity of a functional assessment including repeatedly walking back and forth in the movement performance area 115.

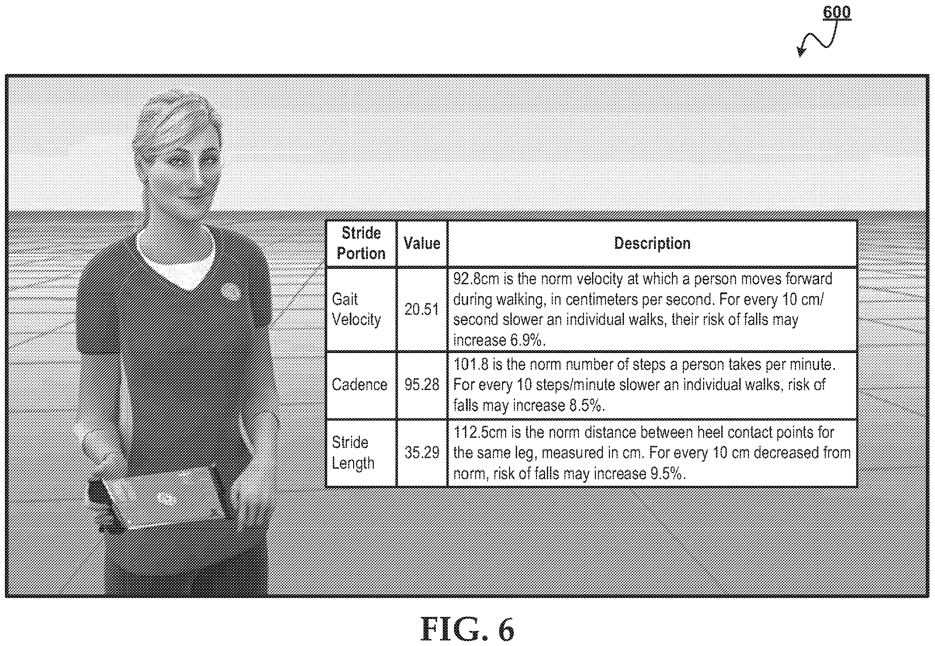

FIG. 6 illustrates an exemplary graphical user interface 600 having functional movement analysis results, which, in various embodiments, are presented to the user 110 via the tablet device 130. The results may include a label of the spatio-temporal gait characteristic, the determined value of the spatio-temporal gait characteristic, and a brief description of the spatio-temporal gait characteristic. The kiosk fall risk assessment system may provide the determined spatio-temporal gait characteristics to the user in real-time.

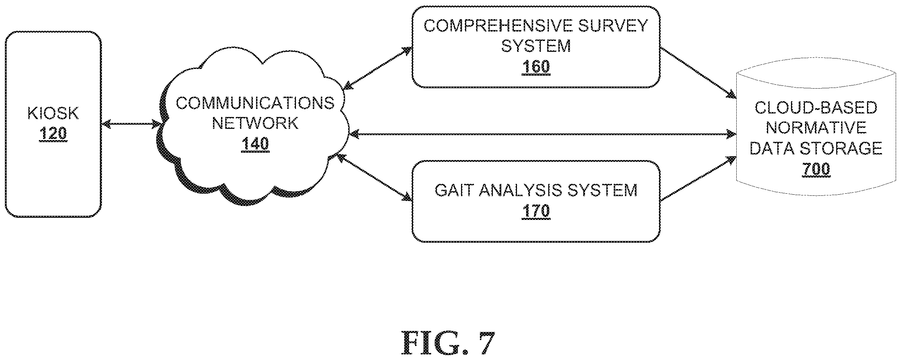

FIG. 7 depicts a cloud-based normative data storage 700 coupled to a portion of the kiosk fall risk assessment system 100 as shown in FIG. 1. The cloud-based normative data storage 700, which may comprise one or more remote servers, provides evidence-based consultative recommendations the user 110 based on the results of the comprehensive fall risk assessment. The one or more remote servers may have computing capabilities as described in the exemplary computing system depicted in FIG. 13. To provide the recommendations following completion of the fall risk assessment, the kiosk does access one or more cloud-based normative data storage 700 instances having evidence-based recommendations for low-risk environmental designs. Furthermore the one or more cloud-based normative data storage 700 instances may also have at least one of: 1) normative data for functional movement tests; 2) known risk ratios for functional movement data; 3) falls history risk ratios; 4) data describing known risks and interactions associated with medication; 5) normative data associate with vision; or 6) known risk ratios associated with vision variables. The normative data and risk ratios may be determined from a plurality of survey responses and functional assessments of other users received by the cloud-based normative data storage 700 from the comprehensive survey system 160 and gait analysis system 170. The kiosk 120 may receive the normative data, risk ratios, and recommendations from the cloud-based normative data storage 700 via communications network 140, and compare the data determined from the comprehensive survey system 160 and gait analysis system 170 for the particular user 110 to the data received from the cloud-based normative data storage 700.
Based on the comparison, the kiosk 120 provides various recommendations to the user 110 based on the comprehensive fall risk assessment. The recommendations facilitate decreasing the fall risk associated with the user 110.

Generally speaking, the kiosk 120 may determine a score for the user regarding a particular domain of the comprehensive survey. More specifically, in the context of the claimed invention, the kiosk determines at least an environment score for the user regarding the environmental domain of the survey. In one or more embodiments, the kiosk fall risk assessment system 100 utilizes one or more artificial intelligence systems and/or machine learning algorithms to determine the score. Generally speaking. The kiosk 120 may further receive a predetermined threshold from the cloud-based normative data storage 700 regarding the particular domain. More specifically, in the context of the claimed invention, the kiosk receives a predetermined threshold associated with the environmental domain of the survey from a cloud-based normative data storage. Generally speaking, upon Upon determining that the score exceeds the predetermined threshold, the kiosk 120 may determine that one or more recommendations received from the cloud-based normative data storage 700 associated with the particular domain are applicable to the user 110 and display the one or more recommendations to the user 110. More specifically, in the context of the claimed invention, upon determining that the environment score exceeds the predetermined threshold associated with the environment domain, the kiosk 120 determines that one or more recommendations associated with the environment domain are applicable to the user 110 and displays the one or more recommendations to the user 110, the one or more recommendations being evidenced-based recommendations for low-risk environmental designs.

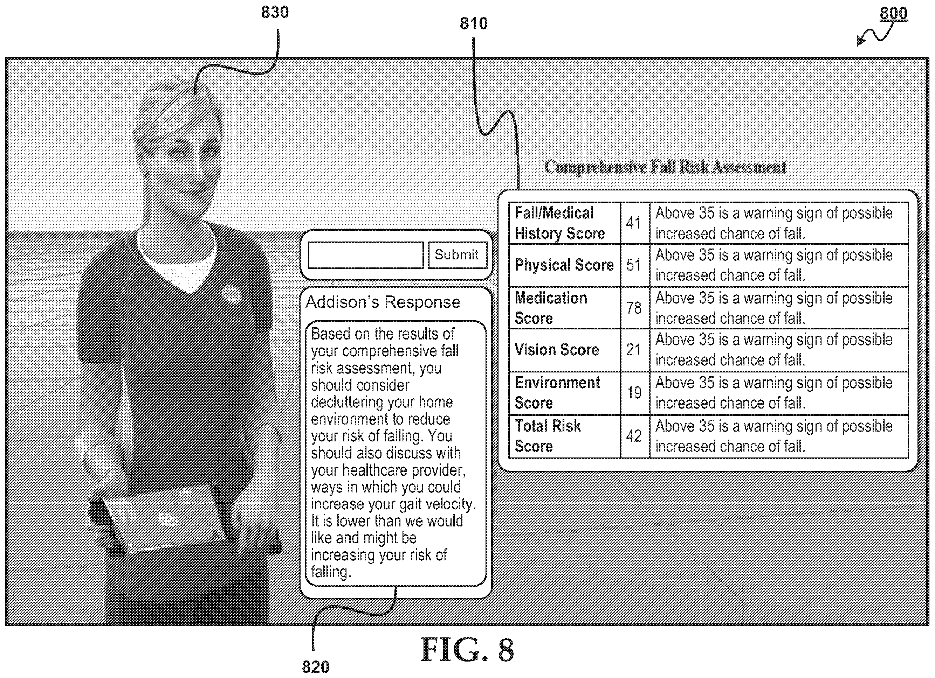

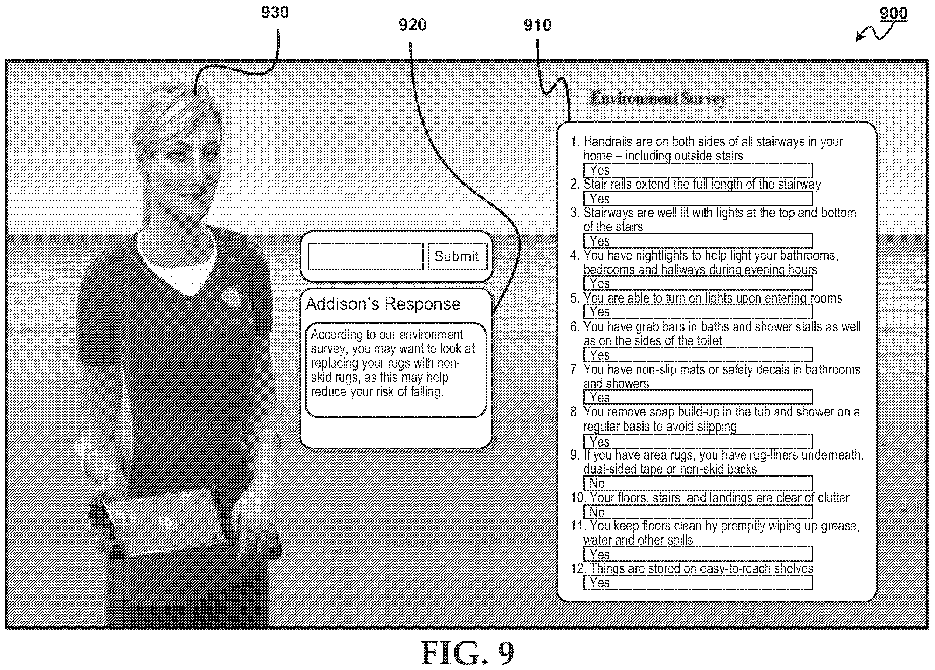

FIGS. 8 and 9 depict providing the user with one or more recommendations based on the comprehensive fall risk assessment. FIG. 8 illustrates a graphical user interface 800 having recommendations 820 determined based on one or more survey responses and determined spatio-temporal gait characteristics presented to the user in a comprehensive fall risk assessment 810. For example, the ECI avatar 830 provides a recommendation 820 that the user should consider decluttering his or her home environment to reduce the risk of falling, based on the environment score present in the user's comprehensive fall risk assessment 810. The ECI avatar 830 likewise recommends that the user discuss with a healthcare provider ways in which he or she could increase gait velocity, for example based on the determined spatio-temporal gait characteristic shown in FIG. 6 which is below a normative value threshold. FIG. 9 shows a graphical user interface 900 having an environment survey 910, recommendations 920, and ECI avatar 930. Based on the results of the environmental survey 910, in particular the user input that the user has area rugs but no rug-liners underneath, dual-sided tape, or non-skid backs, as well as that the user has floors, stairs, and landings not clear of clutter, the ECI avatar 930 provides the recommendation 920 that the user should replace his or her rugs with non-skid rugs to help reduce the risk of falling.

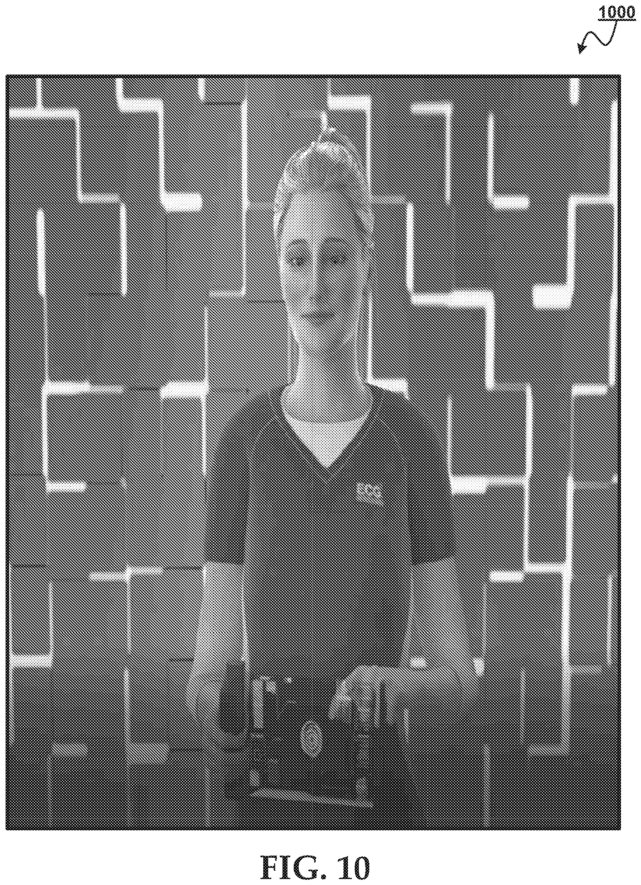

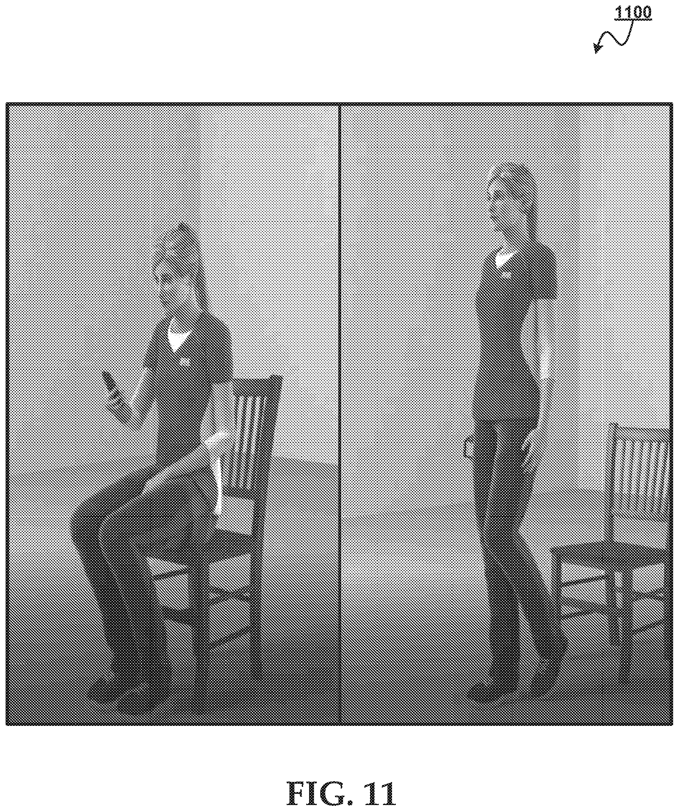

FIG. 10 depicts an exemplary ECI avatar 1000 configured to guide the user through the completion of the comprehensive fall risk assessment. The ECI avatar 1000 facilitates guiding the user 110 through the data collection process in a manner that serves three purposes: 1) to reduce user errors in data collection processes performed by the kiosk 120; 2) to provide an interactive method of click-free and type-free communication that is easily completed by the user 110 and 3) to provide both instructions and representative examples of the various tests to be performed by the user 110. For example, as depicted in FIG. 11, the ECI avatar 1100 may provide a representative example of a functional test to be performed by the user. In particular the ECI avatar 1100 depicted in FIG. 11 is demonstrating how the user should properly transition from a sitting position to a standing position such that the kiosk fall risk assessment system is able to adequately record depth information and properly analyze the activity. As such, the kiosk fall risk assessment system may utilize cloud-based resources coupled to the kiosk to allow survey responses to be received and assessed in a manner that is less intimidating than a more traditional laboratory setting or medical professional's office.

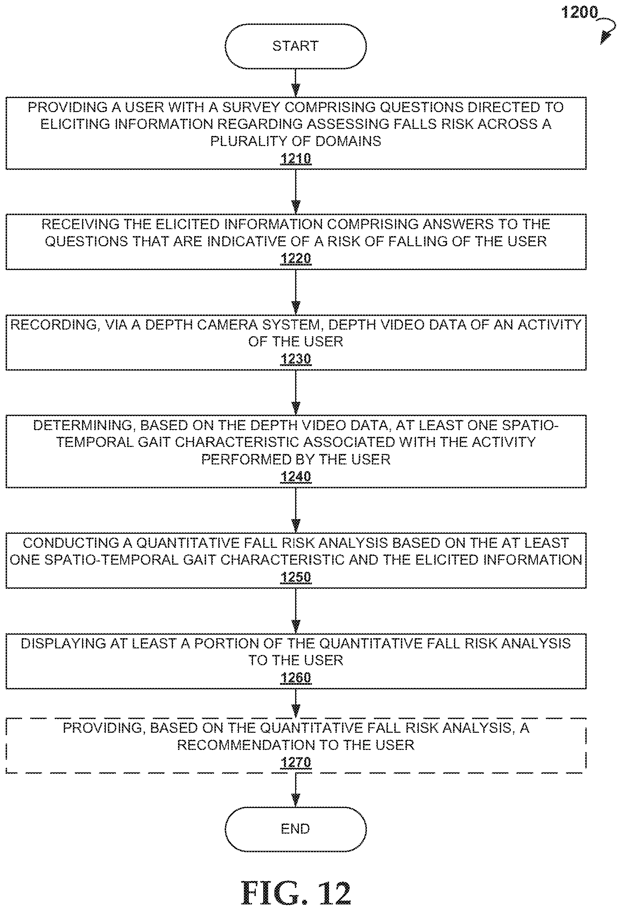

FIG. 12 is a process flow diagram showing a method 1200 for providing a comprehensive fall risk assessment according to one or more example embodiments. The method 1200 may be performed by processing logic that may comprise hardware (e.g., dedicated logic, programmable logic, and microcode), software (such as software run on a general-purpose computer system or a dedicated machine), or a combination thereof. In one or more example embodiments, the processing logic resides at the kiosk 120, the tablet device 130, the comprehensive survey system 160, or the gait analysis system 170, or combinations thereof.

As shown in FIG. 12, the method 1200 does commence at operation 1210, with providing a user with a survey comprising questions directed to eliciting information regarding assessing falls risk across a plurality of domains, including at least an environmental domain. The user may be provided with the survey via kiosk, and more specifically, an ECI avatar represented by a graphical user interface of a coupled tablet device.

At operation 1220, the method 1200 proceeds with receiving the elicited information comprising answers to the questions that are indicative of a risk of falling of the user.

At operation 1230, the method 1200 includes recording, via a depth camera system, depth video data of an activity of the user.

At operation 1240, the method 1200 proceeds with determining, based on the recorded depth video data, at least one spatio-temporal gait characteristic associated with the activity performed by the user. The determining may include tracking body movements via a body tracking module and calculating the spatio-temporal gait characteristic via a movement parameter module, as previously described. It is to be understood that the calculation of the spatio-temporal gait characteristic may be performed by the kiosk or by a remote server such as the gait analysis system 170 depicted in FIG. 1. In this way, the kiosk may determine the at least one spatio-temporal gait characteristic by transmitting the depth video data to such a remote server for processing, and receiving the spatio-temporal gait characteristics from the remote server.

At operation 1250, the method 1200 includes conducting a quantitative fall risk analysis based on the at least one spatio-temporal gait characteristic and the elicited information comprising the answers to the survey questions. The quantitative gait analysis may include determining an overall fall risk score based on a set of spatio-temporal gait characteristics.

At operation 1260, the method 1200 proceeds with displaying at least a portion of the quantitative fall risk analysis to the user.

At operation 1270, the method 1200 includes providing, based on the quantitative fall risk analysis, a recommendation to the user, the recommendation being an evidenced-based recommendation for low-risk environmental designs.

FIG. 13 shows a diagrammatic representation of a computing device for a machine in the example electronic form of a computer system 1300, within which a set of instructions for causing the machine to perform any one or more of the methodologies discussed herein can be executed. In example embodiments, the machine operates as a standalone device, or can be connected (e.g., networked) to other machines. In a networked deployment, the machine can operate in the capacity of a server, a client machine in a server-client network environment, or as a peer machine in a peer-to-peer (or distributed) network environment. The machine can be a personal computer (PC), tablet PC, game console, set-top box (STB), personal digital assistant (PDA), television device, cellular telephone, portable music player (e.g., a portable hard drive audio device), web appliance, or any machine capable of executing a set of instructions (sequential or otherwise) that specify actions to be taken by that machine. Further, while only a single machine is illustrated, the term "machine" shall also be taken to include any collection of machines that separately or jointly execute a set (or multiple sets) of instructions to perform any one or more of the methodologies discussed herein. Computer system 1300 can be an instance of the kiosk 120, tablet device 130, comprehensive survey system 160, gait analysis system 170, or cloud-based normative data storage 700.

The example computer system 1300 includes a processor or multiple processors 1305 (e.g., a central processing unit (CPU), a graphics processing unit (GPU), or both), and a main memory 1310 and a static memory 1315, which communicate with each other via a bus 1320. The computer system 1300 can further include a video display unit 1325 (e.g., a liquid-crystal display (LCD), organic light emitting diode (OLED) display, or a cathode ray tube (CRT)). The computer system 1300 also includes at least one input device 1330, such as an alphanumeric input device (e.g., a keyboard), a cursor control device (e.g., a mouse), a microphone, a digital camera, a video camera, and so forth. The computer system 1300 also includes a disk drive unit 1335, a signal generation device 1340 (e.g., a speaker), and a network interface device 1345.

The drive unit 1335 (also referred to as the disk drive unit 1335) includes a machine-readable medium 1350 (also referred to as a computer-readable medium 1350), which stores one or more sets of instructions and data structures (e.g., instructions 1355) embodying or utilized by any one or more of the methodologies or functions described herein. The instructions 1355 can also reside, completely or at least partially, within the main memory 1310, static memory 1315 and/or within the processor(s) 1305 during execution thereof by the computer system 1300. The main memory 1310, static memory 1315, and the processor(s) 1305 also constitute machine-readable media.

The instructions 1355 can further be transmitted or received over a communications network 1360 via the network interface device 1345 utilizing any one of a number of well-known transfer protocols (e.g., Hyper Text Transfer Protocol (HTTP), CAN, Serial, and Modbus). The communications network 1360 includes the Internet, local intranet, Personal Area Network (PAN) , Local Area Network (LAN), Wide Area Network (WAN), Metropolitan Area Network (MAN), virtual private network (VPN), storage area network (SAN), frame relay connection, Advanced Intelligent Network (AIN) connection, synchronous optical network (SONET) connection, digital T1, T3, E1 or E3 line, Digital Data Service (DDS) connection, Digital Subscriber Line (DSL) connection, Ethernet connection, Integrated Services Digital Network (ISDN) line, cable modem, Asynchronous Transfer Mode (ATM) connection, or an Fiber Distributed Data Interface (FDDI) or Copper Distributed Data Interface (CDDI) connection. Furthermore, communications network 1360 can also include links to any of a variety of wireless networks including Wireless Application Protocol (WAP), General Packet Radio Service (GPRS), Global System for Mobile Communication (GSM), Code Division Multiple Access (CDMA) or Time Division Multiple Access (TDMA), cellular phone networks, Global Positioning System (GPS), cellular digital packet data (CDPD), Research in Motion, Limited (RIM) duplex paging network, Bluetooth radio, or an IEEE 802.11-based radio frequency network.

While the machine-readable medium 1350 is shown in an example embodiment to be a single medium, the term "computer-readable medium" should be taken to include a single medium or multiple media (e.g., a centralized or distributed database, and/or associated caches and servers) that store the one or more sets of instructions. The term "computer-readable medium" shall also be taken to include any medium that is capable of storing, encoding, or carrying a set of instructions for execution by the machine and that causes the machine to perform any one or more of the methodologies of the present application, or that is capable of storing, encoding, or carrying data structures utilized by or associated with such a set of instructions. The term "computer-readable medium" shall accordingly be taken to include, but not be limited to, solid-state memories, optical and magnetic media. Such media can also include, without limitation, hard disks, floppy disks, flash memory cards, digital video disks, random access memory (RAM), read only memory (ROM), and the like.

The example embodiments described herein can be implemented in an operating environment comprising computer-executable instructions (e.g., software) installed on a computer, in hardware, or in a combination of software and hardware. The computer-executable instructions can be written in a computer programming language or can be embodied in firmware logic. If written in a programming language conforming to a recognized standard, such instructions can be executed on a variety of hardware platforms and for interfaces to a variety of operating systems. Although not limited thereto, computer software programs for implementing the present method can be written in any number of suitable programming languages such as, for example, Hypertext Markup Language (HTML), Dynamic HTML, XML, Extensible Stylesheet Language (XSL), Document Style Semantics and Specification Language (DSSSL), Cascading Style Sheets (CSS), Synchronized Multimedia Integration Language (SMIL), Wireless Markup Language (WML), Java^{™}, Jini^{™}, C, C++, C#, .NET, Adobe Flash, Perl, UNIX Shell, Visual Basic or Visual Basic Script, Virtual Reality Markup Language (VRML), ColdFusion^{™} or other compilers, assemblers, interpreters, or other computer languages or platforms.

Thus, the technology for comprehensive fall risk assessment is disclosed. Although embodiments have been described with reference to specific example embodiments, it will be evident that various modifications and changes can be made to these example embodiments without departing from the scope of the invention which is defined by the appended claims. Accordingly, the specification and drawings are to be regarded in an illustrative rather than a restrictive sense.

## Claims

1. A computer-implemented method (1200) for providing real-time assessment of falling risk, the method comprising:
providing (1210) a user with a survey comprising questions directed to eliciting information regarding assessing falls risk across a plurality of domains, the survey comprising an environment survey comprising questions directed to eliciting information regarding assessing falls risk in an environmental domain;
receiving (1220) the elicited information comprising answers to the questions that are indicative of a risk of falling of the user;
recording (1230), via a depth camera system, depth video data of an activity of the user;
determining (1240), based on the depth video data, at least one spatio-temporal gait characteristic associated with the activity performed by the user;
determining an environment score for the user regarding the environmental domain of the survey, the environment score being determined based on the elicited information comprising answers to the questions of the environment survey;
conducting (1250) a quantitative fall risk analysis based on the at least one spatio-temporal gait characteristic and the elicited information and further based on the environment score;
displaying (1260) at least a portion of the quantitative fall risk analysis to the user; and,
providing (1270), in real-time, consultative recommendations based on the quantitative fall risk analysis, wherein providing (1270) the consultative recommendations includes:
receiving a predetermined threshold associated with a domain from a cloud-based normative data storage, wherein the domain is the environment domain;
comparing a domain score of the quantitative fall risk analysis to the predetermined threshold, wherein the domain score is the determined environment score;
determining, based on the comparison, that the domain score exceeds the predetermined threshold; and
displaying, if the domain score exceeds the predetermined threshold, a recommendation to the user, wherein the recommendation is evidenced-based recommendations for low-risk environmental designs.

2. The method as recited in claim 1, wherein the providing the user with the survey includes presenting the questions with an interactive animated conversational graphical user interface.

3. The method as recited in claim 2, further comprising displaying a representation of the activity to be performed by the user via the interactive animated conversational graphical user interface.

4. The method as recited in claim 1, wherein the plurality of domains include history, medication, physical, and vision.

5. The method as recited in claim 1, wherein the determining (1240) the at least one spatio-temporal gait characteristic includes tracking a position and orientation of the user in the depth video data.

6. The method as recited in claim 1, wherein providing (1270) the consultative recommendations includes:
accessing the cloud-based normative data storage having normative data, risk ratios, and recommendations;
comparing the quantitative fall risk analysis to the normative data and risk ratios; and
based on the comparison, selecting a recommendation of the recommendations accessed from the cloud-based normative data storage.

7. A system (100) for providing real-time assessment of falling risk, the system comprising:
a kiosk (120);
a tablet device (130) communicatively coupled to the kiosk (120);
a depth camera system (150) communicatively coupled to the kiosk (120);
at least one processor (1305); and
a memory (1310, 1315) storing processor-executable instructions, wherein the at least one processor is configured to implement the following operations upon executing the processor-executable instructions:
providing (1210) a user with a survey comprising questions directed to eliciting information regarding assessing falls risk across a plurality of domains, the survey comprising an environment survey comprising questions directed to eliciting information regarding assessing falls risk in an environmental domain;
receiving (1220) the elicited information comprising answers to the questions that are indicative of a risk of falling of the user;
recording (1230), via a depth camera system, depth video data of an activity of the user;
determining (1240), based on the depth video data, at least one spatio-temporal gait characteristic associated with the activity performed by the user;
determining an environment score for the user regarding the environmental domain of the survey, the environment score being determined based on the elicited information comprising answers to the questions of the environment survey;
conducting (1250) a quantitative fall risk analysis based on the at least one spatio-temporal gait characteristic and the elicited information and further based on the environment score; and
displaying (1260) at least a portion of the quantitative fall risk analysis to the user,
wherein the at least one processor (1305) is further configured to implement the operation of providing (1270), in real-time, consultative recommendations based on the quantitative fall risk analysis, wherein providing (1270) the consultative recommendations includes:
receiving a predetermined threshold associated with a domain from a cloud-based normative data storage, wherein the domain is the environment domain;
comparing a domain score of the quantitative fall risk analysis to the predetermined threshold, wherein the domain score is the determined environment score;
determining, based on the comparison, that the domain score exceeds the predetermined threshold; and
displaying, if the domain score exceeds the predetermined threshold, a recommendation to the user, wherein the recommendation is evidenced-based recommendations for low-risk environmental designs.

8. The system as recited in claim 7, further comprising an interactive animated conversational graphical user interface displayed by the tablet device (130).

9. The system as recited in claim 8, wherein the at least one processor (1305) is further configured to implement the operation of displaying a representation of the activity to be performed by the user via the interactive animated conversational graphical user interface.

10. The system as recited in claim 7, wherein the determining (1240) the at least one spatio-temporal gait characteristic includes tracking a position and orientation of the user in the depth video data.

11. A non-transitory computer readable medium having embodied thereon instructions being executable by at least one processor (1305) to perform a method for providing real-time assessment of falling risk, the method comprising:
providing (1210) a user with a survey comprising questions directed to eliciting information regarding assessing falls risk across a plurality of domains, the survey comprising an environment survey comprising questions directed to eliciting information regarding assessing falls risk in an environmental domain;
receiving (1220) the elicited information comprising answers to the questions that are indicative of a risk of falling of the user;
recording (1230), via a depth camera system, depth video data of an activity of the user;
determining (1240), based on the depth video data, at least one spatio-temporal gait characteristic associated with the activity performed by the user;
determining an environment score for the user regarding the environmental domain of the survey, the environment score being determined based on the elicited information comprising answers to the questions of the environment survey;
conducting (1250) a quantitative fall risk analysis based on the at least one spatio-temporal gait characteristic and the elicited information and further based on the environment score;
displaying (1260) at least a portion of the quantitative fall risk analysis to the user; and,
providing (1270), in real-time, consultative recommendations based on the quantitative fall risk analysis, wherein providing (1270) the consultative recommendations includes:
receiving a predetermined threshold associated with a domain from a cloud-based normative data storage, wherein the domain is the environment domain;
comparing a domain score of the quantitative fall risk analysis to the predetermined threshold, wherein the domain score is the determined environment score;
determining, based on the comparison, that the domain score exceeds the predetermined threshold; and
displaying, if the domain score exceeds the predetermined threshold, a recommendation to the user, wherein the recommendation is evidenced-based recommendations for low-risk environmental designs.

12. The method as recited in claim 1 or the system as recited in claim 6, wherein the at least one spatio-temporal gait characteristic includes one or more of a gait velocity, a stride length, a step length, and a step width, and/or a time in stance, a time in swing, a time in double support, a stance time variability, and a swing time variability, and/or a test duration, a turning duration, a sit-to-stand duration, a stand-to-sit duration, a number of sit-to-stand repetitions completed within a predetermined period of time, and a number of stand-to-sit repetitions completed within a predetermined period of time.

## Patentansprüche

1. Computer-implementiertes Verfahren (1200) zum Bereitstellen einer Bewertung eines Sturzrisikos in Echtzeit, wobei das Verfahren umfasst:
Bereitstellen (1210), an einen Benutzer, einer Umfrage, die Fragen umfasst, die auf ein Erheben von Informationen bezüglich eines Bewertens eines Risikos von Stürzen über eine Vielzahl von Bereichen gerichtet sind, wobei die Umfrage eine Umgebungsumfrage umfasst, die Fragen umfasst, die auf ein Erheben von Informationen bezüglich eines Bewertens eines Risikos von Stürzen in einem Umgebungsbereich gerichtet sind;
Empfangen (1220) der erhobenen Informationen, die Antworten auf die Fragen umfassen, die auf ein Risiko des Stürzens des Benutzers hinweisen;
Aufzeichnen (1230) von Tiefenvideodaten einer Aktivität des Benutzers mittels eines Tiefenkamerasystems;
Bestimmen (1240), auf Grundlage der Tiefenvideodaten, mindestens einer räumlich-zeitlichen Gangcharakteristik, die der vom Benutzer ausgeführten Aktivität zugeordnet ist;
Bestimmen einer Umgebungspunktzahl für den Benutzer bezüglich des Umgebungsbereichs der Umfrage, wobei die Umgebungspunktzahl auf Grundlage der erhobenen Information bestimmt wird, die Antworten auf die Fragen der Umgebungsumfrage umfasst;
Durchführen (1250) einer quantitativen Sturzrisikoanalyse auf Grundlage der mindestens einen räumlich-zeitlichen Gangcharakteristik und der erhobenen Informationen und ferner auf Grundlage der Umgebungspunktzahl;
Anzeigen (1260) mindestens eines Teils der quantitativen Sturzrisikoanalyse an den Benutzer; und
Bereitstellen (1270) beratender Empfehlungen auf Grundlage der quantitativen Sturzrisikoanalyse in Echtzeit, wobei das Bereitstellen (1270) der beratenden Empfehlungen beinhaltet:
Empfangen eines vorbestimmten Schwellwertes, der einem Bereich aus einem cloudbasierten normativen Datenspeicher zugeordnet ist, wobei der Bereich der Umgebungsbereich ist;
Vergleichen einer Bereichspunktzahl der quantitativen Sturzrisikoanalyse mit dem vorbestimmten Schwellwert, wobei die Bereichspunktzahl die bestimmte Umgebungspunktzahl ist;
Ermitteln, auf Grundlage des Vergleichs, dass die Umgebungspunktzahl den vorbestimmten Schwellwert übersteigt; und
Anzeigen, falls die Umgebungspunktzahl den vorbestimmten Schwellwert übersteigt, einer Empfehlung an den Benutzer, wobei die Empfehlung evidenzbasierte Empfehlungen für risikoarme Umgebungsgestaltungen darstellt.

2. Verfahren nach Anspruch 1, wobei das Bereitstellen der Umfrage an den Benutzer ein Präsentieren der Fragen mit einer interaktiven, animierten, dialogfähigen grafischen Benutzeroberfläche beinhaltet.

3. Verfahren nach Anspruch 2, das ferner ein Anzeigen einer Darstellung der von dem Benutzer auszuführenden Aktivität über die interaktive, animierte, dialogfähige grafische Benutzeroberfläche umfasst.

4. Verfahren nach Anspruch 1, wobei die Vielzahl von Bereichen Vorgeschichte, Medikation, Körperliches und Sehvermögen beinhaltet.

5. Verfahren nach Anspruch 1, wobei das Bestimmen (1240) der mindestens einen räumlich-zeitlichen Gangcharakteristik ein Nachverfolgen der Position und Ausrichtung des Benutzers in den Tiefenvideodaten beinhaltet.

6. Verfahren nach Anspruch 1, wobei das Bereitstellen (1270) der beratenden Empfehlungen beinhaltet:
Zugreifen auf den cloudbasierten normativen Datenspeicher mit normativen Daten, Risikokennzahlen und Empfehlungen;
Vergleichen der quantitativen Sturzrisikoanalyse mit den normativen Daten und Risikokennzahlen; und
Auswählen, auf Grundlage des Vergleichs, einer Empfehlung aus den Empfehlungen, auf die aus dem cloudbasierten normativen Datenspeicher zugegriffen wird.

7. System (100) zum Bereitstellen einer Bewertung eines Sturzrisikos in Echtzeit,
wobei das System umfasst:
einen Kiosk (120);
ein Tablet-Gerät (130), das mit dem Kiosk (120) kommunikativ gekoppelt ist;
ein Tiefenkamerasystem (150), das mit dem Kiosk (120) kommunikativ gekoppelt ist; mindestens einen Prozessor (1305); und
einen Speicher (1310, 1315), der vom Prozessor ausführbare Anweisungen speichert, wobei der mindestens eine Prozessor konfiguriert ist, um bei der Ausführung der vom Prozessor ausführbaren Anweisungen folgende Operationen durchzuführen:
Bereitstellen (1210), an einen Benutzer, einer Umfrage, die Fragen umfasst, die auf ein Erheben von Informationen bezüglich eines Bewertens eines Risikos von Stürzen über eine Vielzahl von Bereichen gerichtet sind, wobei die Umfrage eine Umgebungsumfrage umfasst, die Fragen umfasst, die auf ein Erheben von Informationen bezüglich eines Bewertens eines Risikos von Stürzen in einem Umgebungsbereich gerichtet sind;
Empfangen (1220) der erhobenen Informationen, die Antworten auf die Fragen umfassen, die auf ein Risiko des Stürzens des Benutzers hinweisen;
Aufzeichnen (1230) von Tiefenvideodaten einer Aktivität des Benutzers mittels eines Tiefenkamerasystems;
Bestimmen (1240), auf Grundlage der Tiefenvideodaten, mindestens einer räumlich-zeitlichen Gangcharakteristik, die der vom Benutzer ausgeführten Aktivität zugeordnet ist;
Bestimmen einer Umgebungspunktzahl für den Benutzer bezüglich des Umgebungsbereichs der Umfrage, wobei die Umgebungspunktzahl auf Grundlage der erhobenen Information bestimmt wird, die Antworten auf die Fragen der Umgebungsumfrage umfasst;
Durchführen (1250) einer quantitativen Sturzrisikoanalyse auf Grundlage der mindestens einen räumlich-zeitlichen Gangcharakteristik und der erhobenen Informationen und ferner auf Grundlage der Umgebungspunktzahl; und
Anzeigen (1260) mindestens eines Teils der quantitativen Sturzrisikoanalyse an den Benutzer; wobei der mindestens eine Prozessor (1305) ferner konfiguriert ist, um die Operation zum Bereitstellen (1270) beratender Empfehlungen auf Grundlage der quantitativen Sturzrisikoanalyse in Echtzeit durchzuführen, wobei das Bereitstellen (1270) der beratenden Empfehlungen beinhaltet:
Empfangen eines vorbestimmten Schwellwertes, der einem Bereich aus einem cloudbasierten normativen Datenspeicher zugeordnet ist, wobei der Bereich der Umgebungsbereich ist;
Vergleichen einer Bereichspunktzahl der quantitativen Sturzrisikoanalyse mit dem vorbestimmten Schwellwert, wobei die Bereichspunktzahl die bestimmte Umgebungspunktzahl ist;
Bestimmen, auf Grundlage des Vergleichs, dass die Umgebungspunktzahl den vorbestimmten Schwellwert übersteigt; und
Anzeigen, falls die Umgebungspunktzahl den vorbestimmten Schwellwert übersteigt, einer Empfehlung an den Benutzer, wobei die Empfehlung evidenzbasierte Empfehlungen für risikoarme Umgebungsgestaltungen darstellt.

8. System nach Anspruch 7, das ferner eine interaktive, animierte, dialogfähige grafische Benutzeroberfläche umfasst, die von dem Tablet-Gerät (130) angezeigt wird.

9. System nach Anspruch 8, wobei der mindestens eine Prozessor (1305) ferner konfiguriert ist, um die Operation eines Anzeigens einer Darstellung der von dem Benutzer auszuführenden Aktivität über die interaktive, animierte, dialogfähige grafische Benutzeroberfläche durchzuführen.

10. System nach Anspruch 7, wobei das Bestimmen (1240) der mindestens einen räumlich-zeitlichen Gangcharakteristik ein Nachverfolgen der Position und Ausrichtung des Benutzers in den Tiefenvideodaten beinhaltet.

11. Nicht-flüchtiges computerlesbares Medium mit darauf verankerten Anweisungen, die von mindestens einem Prozessor (1305) ausgeführt werden können, um ein Verfahren zum Bereitstellen einer Bewertung eines Sturzrisikos in Echtzeit auszuführen, wobei das Verfahren umfasst:
Bereitstellen (1210), an einen Benutzer, einer Umfrage, die Fragen umfasst, die auf ein Erheben von Informationen bezüglich eines Bewertens eines Risikos von Stürzen über eine Vielzahl von Bereichen gerichtet sind, wobei die Umfrage eine Umgebungsumfrage umfasst, die Fragen umfasst, die auf ein Erheben von Informationen bezüglich eines Bewertens eines Risikos von Stürzen in einem Umgebungsbereich gerichtet sind;
Empfangen (1220) der erhobenen Informationen, die Antworten auf die Fragen umfassen, die auf ein Risiko des Stürzens des Benutzers hinweisen;
Aufzeichnen (1230) von Tiefenvideodaten einer Aktivität des Benutzers mittels eines Tiefenkamerasystems;
Bestimmen (1240), auf Grundlage der Tiefenvideodaten, mindestens einer räumlich-zeitlichen Gangcharakteristik, die der vom Benutzer ausgeführten Aktivität zugeordnet ist;
Bestimmen einer Umgebungspunktzahl für den Benutzer bezüglich des Umgebungsbereichs der Umfrage, wobei die Umgebungspunktzahl auf Grundlage der erhobenen Information bestimmt wird, die Antworten auf die Fragen der Umgebungsumfrage umfasst;
Durchführen (1250) einer quantitativen Sturzrisikoanalyse auf Grundlage der mindestens einen räumlich-zeitlichen Gangcharakteristik und der erhobenen Informationen und ferner auf Grundlage der Umgebungspunktzahl;
Anzeigen (1260) mindestens eines Teils der quantitativen Sturzrisikoanalyse an den Benutzer; und
Bereitstellen (1270) beratender Empfehlungen auf Grundlage der quantitativen Sturzrisikoanalyse in Echtzeit, wobei das Bereitstellen (1270) der beratenden Empfehlungen Folgendes beinhaltet:
Empfangen eines vorbestimmten Schwellwertes, der einem Bereich aus einem cloudbasierten normativen Datenspeicher zugeordnet ist, wobei der Bereich der Umgebungsbereich ist;
Vergleichen einer Bereichspunktzahl der quantitativen Sturzrisikoanalyse mit dem vorbestimmten Schwellwert, wobei die Bereichspunktzahl die bestimmte Umgebungspunktzahl ist;
Bestimmen, auf Grundlage des Vergleichs, dass die Umgebungspunktzahl den vorbestimmten Schwellwert übersteigt; und
Anzeigen, falls die Umgebungspunktzahl den vorbestimmten Schwellwert übersteigt, einer Empfehlung an den Benutzer, wobei die Empfehlung evidenzbasierte Empfehlungen für risikoarme Umgebungsgestaltungen darstellt.

12. Verfahren nach Anspruch 1 oder System nach Anspruch 6, wobei die mindestens eine räumlich-zeitliche Gangcharakteristik eines oder mehrere beinhaltet aus: eine Ganggeschwindigkeit, eine Schrittweite, eine Schrittlänge und eine Schrittbreite und/oder eine Zeit im Stand, eine Zeit im Schwung, eine Zeit im Doppelstütz, eine Standzeitvariabilität und eine Schwungzeitvariabilität und/oder eine Testdauer, eine Drehdauer, eine Sitz-zu-Steh-Dauer, eine Steh-zu-Sitz-Dauer, eine Anzahl von Sitz-zu-Steh-Wiederholungen, die innerhalb einer vorbestimmten Zeitspanne durchgeführt werden, und eine Anzahl von Steh-zu-Sitz-Wiederholungen, die innerhalb einer vorbestimmten Zeitspanne durchgeführt werden.

## Revendications

1. Procédé mis en oeuvre par ordinateur (1200) pour fournir une évaluation en temps réel de risque de chute, le procédé comprenant les étapes ci-dessous consistant à :
fournir (1210), à un utilisateur, une enquête comprenant des questions visant à obtenir des informations concernant l'évaluation d'un risque de chute dans une pluralité de domaines, l'enquête comprenant une enquête d'environnement comprenant des questions visant à obtenir des informations sur l'évaluation d'un risque de chute dans un domaine environnemental ;
recevoir (1220) les informations obtenues comprenant des réponses aux questions qui sont indicatives d'un risque de chute de l'utilisateur ;
enregistrer (1230), par l'intermédiaire d'un système de caméra de profondeur, des données vidéo de profondeur d'une activité de l'utilisateur ;
déterminer (1240), sur la base des données vidéo de profondeur, au moins une caractéristique de marche spatio-temporelle associée à l'activité mise en oeuvre par l'utilisateur ;
déterminer un score d'environnement pour l'utilisateur concernant le domaine environnemental de l'enquête, le score d'environnement étant déterminé sur la base des informations obtenues comprenant des réponses aux questions de l'enquête d'environnement ;
effectuer (1250) une analyse quantitative de risque de chute sur la base de ladite au moins une caractéristique de marche spatio-temporelle et des informations obtenues, ainsi que sur la base du score d'environnement ;
afficher (1260) au moins une partie de l'analyse quantitative de risque de chute à l'utilisateur ; et
fournir (1270), en temps réel, des recommandations consultatives basées sur l'analyse quantitative de risque de chute, dans laquelle l'étape de fourniture (1270) des recommandations consultatives inclut les étapes ci-dessous consistant à :
recevoir un seuil prédéterminé associé à un domaine à partir d'un magasin de stockage de données normatives basé sur l'informatique en nuage, dans lequel le domaine est le domaine d'environnement ;
comparer un score de domaine de l'analyse quantitative de risque de chute au seuil prédéterminé, dans lequel le score de domaine est le score d'environnement déterminé ;
déterminer, sur la base de la comparaison, que le score de domaine dépasse le seuil prédéterminé ; et
afficher, si le score de domaine dépasse le seuil prédéterminé, une recommandation à l'utilisateur, dans laquelle la recommandation correspond à des recommandations fondées sur des preuves pour des conceptions environnementales à faible risque.

2. Procédé selon la revendication 1, dans lequel l'étape de fourniture de l'enquête à l'utilisateur inclut l'étape consistant à présenter les questions avec une interface utilisateur graphique conversationnelle animée interactive.

3. Procédé selon la revendication 2, comprenant en outre l'étape consistant à afficher une représentation de l'activité devant être mise en oeuvre par l'utilisateur par l'intermédiaire de l'interface utilisateur graphique conversationnelle animée interactive.

4. Procédé selon la revendication 1, dans lequel la pluralité de domaines inclut les domaines ci-après : antécédents, traitements médicaux, activité physique et vision.

5. Procédé selon la revendication 1, dans lequel l'étape de détermination (1240) de ladite au moins une caractéristique de marche spatio-temporelle inclut l'étape consistant à suivre une position et une orientation de l'utilisateur dans les données vidéo de profondeur.

6. Procédé selon la revendication 1, dans lequel l'étape de fourniture (1270) des recommandations consultatives inclut les étapes ci-dessous consistant à :
accéder au magasin de stockage de données normatives basé sur l'informatique en nuage présentant des données normatives, des ratios de risque et des recommandations ;
comparer l'analyse quantitative de risque de chute aux données normatives et aux ratios de risque ; et
sur la base de la comparaison, sélectionner une recommandation parmi les recommandations accessibles à partir du magasin de stockage de données normatives basé sur l'informatique en nuage.

7. Système (100) destiné à fournir une évaluation en temps réel de risque de chute, le système comprenant :
un kiosque (120) ;
un dispositif de tablette (130) couplé en communication au kiosque (120) ;
un système de caméra de profondeur (150) couplé en communication au kiosque (120) ;
au moins un processeur (1305) ; et
une mémoire (1310, 1315) stockant des instructions exécutables par processeur, dans lequel ledit au moins un processeur est configuré de manière à mettre en oeuvre les opérations suivantes, suite à l'exécution des instructions exécutables par processeur, consistant à :
fournir (1210), à un utilisateur, une enquête comprenant des questions visant à obtenir des informations concernant l'évaluation d'un risque de chute dans une pluralité de domaines, l'enquête comprenant une enquête d'environnement comprenant des questions visant à obtenir des informations sur l'évaluation d'un risque de chute dans un domaine environnemental ;
recevoir (1220) les informations obtenues comprenant des réponses aux questions qui sont indicatives d'un risque de chute de l'utilisateur ;
enregistrer (1230), par l'intermédiaire d'un système de caméra de profondeur, des données vidéo de profondeur d'une activité de l'utilisateur ;
déterminer (1240), sur la base des données vidéo de profondeur, au moins une caractéristique de marche spatio-temporelle associée à l'activité mise en oeuvre par l'utilisateur ;
déterminer un score d'environnement pour l'utilisateur concernant le domaine environnemental de l'enquête, le score d'environnement étant déterminé sur la base des informations obtenues comprenant des réponses aux questions de l'enquête d'environnement ;
effectuer (1250) une analyse quantitative de risque de chute sur la base de ladite au moins une caractéristique de marche spatio-temporelle et des informations obtenues, ainsi que sur la base du score d'environnement ;
afficher (1260) au moins une partie de l'analyse quantitative de risque de chute à l'utilisateur ;
dans lequel ledit au moins un processeur (1305) est en outre configuré de manière à mettre en oeuvre l'opération consistant à fournir (1270), en temps réel, des recommandations consultatives basées sur l'analyse quantitative de risque de chute, dans laquelle l'étape de fourniture (1270) des recommandations consultatives inclut les étapes ci-dessous consistant à :
recevoir un seuil prédéterminé associé à un domaine à partir d'un magasin de stockage de données normatives basé sur l'informatique en nuage, dans lequel le domaine est le domaine d'environnement ;
comparer un score de domaine de l'analyse quantitative de risque de chute au seuil prédéterminé, dans lequel le score de domaine est le score d'environnement déterminé ;
déterminer, sur la base de la comparaison, que le score de domaine dépasse le seuil prédéterminé ; et
afficher, si le score de domaine dépasse le seuil prédéterminé, une recommandation à l'utilisateur, dans laquelle la recommandation correspond à des recommandations fondées sur des preuves pour des conceptions environnementales à faible risque.

8. Système selon la revendication 7, comprenant en outre une interface utilisateur graphique conversationnelle animée interactive affichée par le dispositif de tablette (130).

9. Système selon la revendication 8, dans lequel ledit au moins un processeur (1305) est en outre configuré de manière à mettre en oeuvre l'opération consistant à afficher une représentation de l'activité devant être mise en oeuvre par l'utilisateur par l'intermédiaire de l'interface utilisateur graphique conversationnelle animée interactive.

10. Système selon la revendication 7, dans lequel l'étape de détermination (1240) de ladite au moins une caractéristique de marche spatio-temporelle inclut l'étape consistant à suivre une position et une orientation de l'utilisateur dans les données vidéo de profondeur.

11. Support non transitoire lisible par ordinateur dans lequel sont incorporées des instructions exécutables par au moins un processeur (1305) pour mettre en oeuvre un procédé de fourniture d'une évaluation en temps réel de risque de chute, le procédé comprenant les étapes ci-dessous consistant à :
fournir (1210), à un utilisateur, une enquête comprenant des questions visant à obtenir des informations concernant l'évaluation d'un risque de chute dans une pluralité de domaines, l'enquête comprenant une enquête d'environnement comprenant des questions visant à obtenir des informations sur l'évaluation d'un risque de chute dans un domaine environnemental ;
recevoir (1220) les informations obtenues comprenant des réponses aux questions qui sont indicatives d'un risque de chute de l'utilisateur ;
enregistrer (1230), par l'intermédiaire d'un système de caméra de profondeur, des données vidéo de profondeur d'une activité de l'utilisateur ;
déterminer (1240), sur la base des données vidéo de profondeur, au moins une caractéristique de marche spatio-temporelle associée à l'activité mise en oeuvre par l'utilisateur ;
déterminer un score d'environnement pour l'utilisateur concernant le domaine environnemental de l'enquête, le score d'environnement étant déterminé sur la base des informations obtenues comprenant des réponses aux questions de l'enquête d'environnement ;
effectuer (1250) une analyse quantitative de risque de chute sur la base de ladite au moins une caractéristique de marche spatio-temporelle et des informations obtenues, ainsi que sur la base du score d'environnement ;
afficher (1260) au moins une partie de l'analyse quantitative de risque de chute à l'utilisateur ; et
fournir (1270), en temps réel, des recommandations consultatives basées sur l'analyse quantitative de risque de chute, dans laquelle l'étape de fourniture (1270) des recommandations consultatives inclut les étapes ci-dessous consistant à :
recevoir un seuil prédéterminé associé à un domaine à partir d'un magasin de stockage de données normatives basé sur l'informatique en nuage, dans lequel le domaine est le domaine d'environnement ;
comparer un score de domaine de l'analyse quantitative de risque de chute au seuil prédéterminé, dans lequel le score de domaine est le score d'environnement déterminé ;
déterminer, sur la base de la comparaison, que le score de domaine dépasse le seuil prédéterminé ; et
afficher, si le score de domaine dépasse le seuil prédéterminé, une recommandation à l'utilisateur, dans laquelle la recommandation correspond à des recommandations fondées sur des preuves pour des conceptions environnementales à faible risque.

12. Procédé selon la revendication 1 ou système selon la revendication 6, dans lequel ladite au moins une caractéristique de marche spatio-temporelle inclut une ou plusieurs des caractéristiques ci-après : une vitesse de marche, une longueur de foulée, une longueur de pas, et une largeur de pas, et/ou un temps d'appui, un temps de balancement, un temps de double appui, une variabilité de temps d'appui, et une variabilité de temps de balancement, et/ou une durée de test, une durée de rotation, une durée de passage d'une position assise à une position debout, une durée de passage d'une position debout à une position assise, un nombre de répétitions de passage d'une position assise à une position debout effectuées au cours d'une période de temps prédéterminée, et un nombre de répétitions de passage d'une position debout à une position assise effectuées au cours d'une période de temps prédéterminée.
